# EUROPÄISCHE PATENTANMELDUNG

(11) **EP 2 258 693 A1**
(43) Veröffentlichungstag der Anmeldung: **08.12.2010**
(21) Anmeldenummer: 10178611.9
(22) Anmeldetag: 20.06.2003
(51) Int. Cl.: C07D 277/36

(54) **Verfahren zum herstellen von Heterocyclischen Fluoralkenylsulfonen**

(30) Priorität: 03.07.2002 DE 10229776
(62) Teilanmeldung aus: 03738072.2
(71) Anmelder: Makhteshim Chemical Works Ltd., 84100 Beer Sheva (IL)
(72) Erfinder: Straub, Alexander, 42117, Wuppertal (DE)
(74) Vertreter: Modiano, Micaela Nadia

(57) **Zusammenfassung**

Die vorliegende Erfindung betrifft ein Verfahren zur Herstellung von hetcrocyctischen Fluoralkenylsulfonen und Fluoralkenylsulfoxiden, indem man die entsprechenden Fluoralkenylthioether mit einem Salz der Peroxomonoschwefelsäure, H₂SO₅, gegebenenfalls in Gegenwart eines Reaktionshilfsmittels und gegebenenfalls in Gegenwart eines Verdünnungsmittels reagieren lässt.

## Beschreibung

Die vorliegende Erfindung betrifft ein Verfahren zur Herstellung von heterocyclischen Fluoralkenylsulfonen und Fluoralkenylsulfoxiden aus den entsprechenden Fluoralkenylthioethern.

Heterocyclische Fluoralkenylsulfone wie z.B. 5-Chlor-2-[(3,4,4-trifluor-3-butenyl)-sulfonyl]-1,3-thiazol, die als Schädlingbekämpfungsmittel z.B. in WO 01/02378, US 3,513,172, US 3,697,538 oder WO 95/24403 beschrieben werden, wurden bislang in der Regel durch die Oxidation des Thioethers mit Wasserstoffperoxid/Eisessig bei 55-60°C/6h in 75%iger Ausbeute hergestellt (vgl. z.B. WO 01/02378). Die Oxidation des Sulfids zum Sulfon verläuft dabei in zwei Schritten. In einem ersten Schritt wird das Sulfid (der Thioether) zum Sulfoxid oxidiert. Im zweiten Schritt wird wiederum das Sulfoxid zum Sulfon oxidiert.

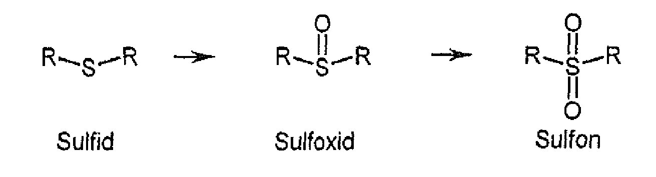

Die Oxidation des Sulfids zum Sulfoxid mit den bekannten Verfahren erfolgt dabei relativ leicht. Die weitere Oxidation des Sulfoxids zum Sulfon geht dagegen nur langsam und schleppend vonstatten und benötigt energischere Bedingungen wie z.B. erhöhte Temperatur und verlängerte Reaktionszeiten, wobei es infolge von Zersetzungs- und Nebenreaktionen zu deutlichen und unerwünschten Ausbeuteeinbußen kommen kann.

Die Verwendung der bisher bekannten, üblichen Oxidationsverfahren mit Wasserstoffperoxid sowie gegebenenfalls Ameisen- oder Essigsäure gegebenenfalls bei Anwesenheit von Molybdat- oder Wolframatkatalysatoren (EP-A1-0 926 143; US 5,965,737; US 6,031,108; Tetrahedron (2001), 57,2469; Spec. Publ. - R. Soc. Chem. (2001), 260, 47) führte in der Regel insbesondere zur unerwünschten Oxidation der Doppelbindung des halogenierten Butenylrestes und zu dessen oxidativem Abbau. Darüberhinaus bergen sie erhebliche Explosionsrisiken.

Aufgabe der vorliegenden Erfindung war es deshalb, ein Verfahren zur oxidativen Darstellung von halogenierten 2-(3-butenylsulfonyl)-1,3-thiazolen aus ihren Sulfiden zur Verfügung zu stellen, welches die Ausbeuteeinbußen durch Zersetzungs- und Nebenreaktionen wie z.B. der Oxidation der Doppelbindung des Butenylrestes vermeidet und des Weiteren ein Verfahren zur Herstellung der erwünschten Verbindungen zu entwickeln, das nicht mit Sicherheitsrisiken verbunden ist.

Überraschenderweise wurde nun gefunden, dass die Verwendung von Salzen der Peroxomonoschwefelsäure der Formel H₂SO₅, wie z.B. Kaliumhydrogenperoxomonosulfat, KHSO₅ als Oxidationsmittel die Verwendung besonders milder Bedingungen erlaubt. Als besonders geeignet haben sich z.B. Oxone® und Caroat® erwiesen. Weiterhin ist als überraschend anzusehen, dass der zweite Schritt der Oxidation des Sulfoxids zum Sulfon nach Neutralisation der Mischung genauso problemlos abläuft wie der erste. Als unerwartet ist insbesondere zu bezeichnen, dass keine nennenswerte Oxidation der Doppelbindung des Trifluorbutenylrestes beobachtet werden konnte, was bei den bekannten Verfahren besonders problematisch ist.

Gegenstand der vorliegenden Erfindung ist deshalb ein Verfahren zur Herstellung von Verbindungen der der Formel (I), 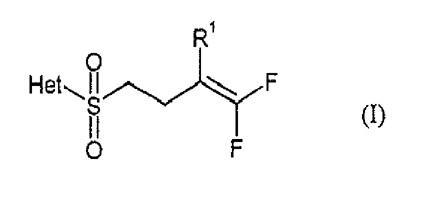 worin
- R¹: für Wasserstoff oder Fluor steht, und
- Het: für einen Heterocyclus aus der nachfolgenden Gruppe von Heterocyclen steht: 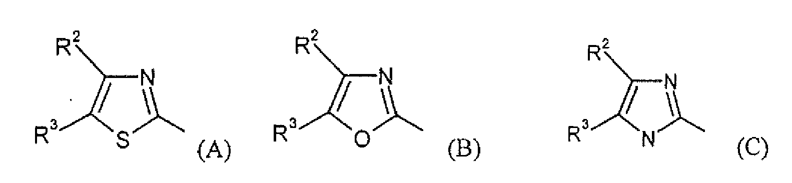 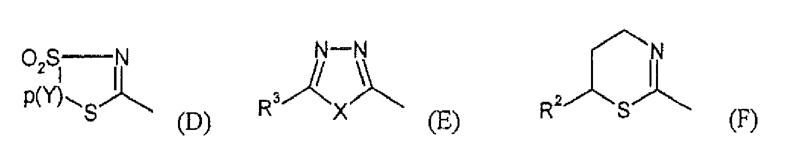 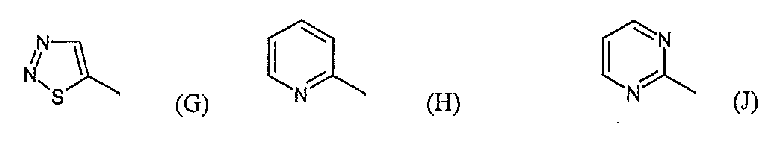 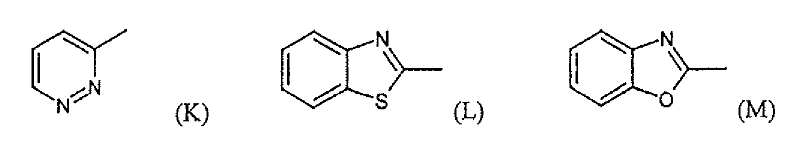 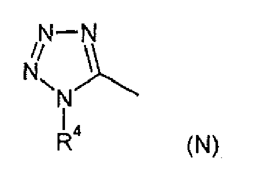
worin
- R²: für Wasserstoff, Halogen, C₁-C₄-Alkyl oder C₁-C₄-Halogenalkyl steht,
- R³: für Wasserstoff, Halogen, sowie jeweils gegebenenfalls durch Halogen, Methyl, Ethyl, n- oder i-Propyl oder n-, i-, s- oder t-Butyl, Methoxy, Ethoxy, n- oder i-Propoxy oder n-, i-, s- oder t-Butoxy substituiertes C₁-C₄-Alkyl, C₁-C₄Alkoxy, C₁-C₄-Alkylthio, C₁-C₄- Alkylsulfinyl, C₁-C₄-Alkylsulfonyl, C₁-C₄-Alkoxy-carbonyl, C₁-C₄- Alkoxy-C₁-C₄-alkyl, C₁-C₄-Alkylthio-C₁-C₄-alkyl, Carboxy, C₁-C₄- Alkylamino-carbonyl, C₃-C₆-Cycloalkyl-aminocarbonyl, C₁-C₄- Dialkyl-aminocarbonyl, C₂-C₄-Alkenyl, C₂-C₄-Alkenylthio, C₂-C₄- Alkenylsulfinyl oder C₂-C₄-Alkenylsulfonyl steht,
- R⁴: für C₁-C₈-Alkyl, C₂-C₆-Alkenyl, C₁-C₄-Haloalkyl, C₁-C₄-Alkoxy- C₁-C₄-alkyl, C₁-C₄-Alkylthio-C₁-C₄-alkyl, C₃-C₈-Cycloalkyl oder für jeweils gegebenenfalls durch Halogen, C₁-C₄-Alkyl, C₁-C₄-Alkoxy, C₁-C₄-Alkylthio oder C₁-C₄-Haloalkyl substituiertes Phenyl oder Benzyl steht,
- p: für 1, 2 oder 3 steht,
- X: für Sauerstoff oder Schwefel steht, und
- Y: für gegebenenfalls einfach oder zweifach, gleich oder verschieden substituiertes Methylen steht, wobei als Substituenten beispielhaft ge- nannt seien: jeweils gegebenenfalls durch Halogen, C₁-C₄-Alkoxy, C₁- C₄-Alkylthio, C₁-C₄- Halogenalkoxy oder C₁-C₄-Halogenalkylthio substituiertes C₁-C₄-Alkyl, C₂-C₄-Alkenyl oder C₂-C₄-Alkinyl, sowie für gegebenenfalls einfach bis dreifach, gleich oder verschieden substituiertes Phenyl, wobei als Substituenten beispielhaft genannt seien: Halogen, Cyano, Nitro, C₁-C₄-Alkyl, C₁-C₄-Alkoxy, C₁-C₄- Alkylthio, C₁-C₄-Halogenalkyl, C₁-C₄-Halogenalkoxy oder C₁-C₄- Halogenalkylthio,
indem man
eine Verbindung der Formel (III), 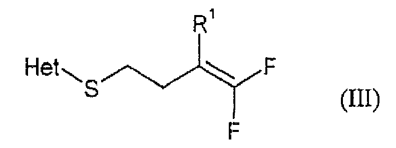 worin
R¹ und Het die vorstehend genannte Bedeutung haben,
mit einem Salz der Peroxomonoschwefelsäure, H₂SO₅,
gegebenenfalls in Gegenwart eines Reaktionshilfsmittels und gegebenenfalls in Gegenwart eines Verdünnungsmittels reagieren lässt.

Bevorzugte Definitionen der Verbindungen der Formel (I), die mit dem erfindungsgemäßen Verfahren hergestellt werden, werden nachfolgend genannt:
- R¹: steht bevorzugt für Fluor.
- Het: steht bevorzugt für einen Heterocyclus aus der nachfolgenden Gruppe von Heterocyclen:

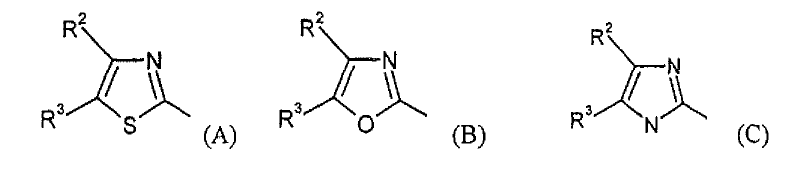

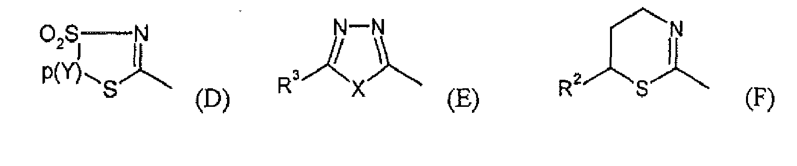

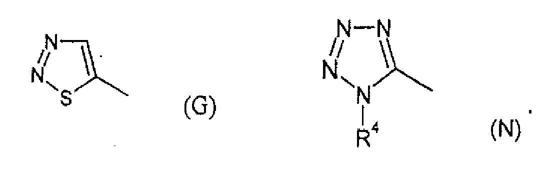

- R²: steht bevorzugt für Wasserstoff, Fluor oder Chlor.
- R³: steht bevorzugt für Wasserstoff, Fluor, Chlor, sowie jeweils gegebenenfalls durch Fluor, Chlor, Methyl, Ethyl, n- oder i-Propyl oder n-, i-, s- oder t-Butyl, Methoxy, Ethoxy, n- oder i-Propoxy öder n-, i-, s- oder t-Butoxy substi- tuiertes Methyl, Ethyl, n- oder i-Propyl, n-, i-, s- oder t-Butyl, Methoxy, Eth- oxy, n- oder i-Propoxy, n-, i-, s- oder t-Butoxy, Methylthio, Ethylthio, n-, oder i-Propylthio, n-, i-, s- oder t-Butylthio, Methylsulfinyl, Ethylsulfinyl, Methylsulfonyl, Ethylsulfonyl, Methoxycarbonyl, Ethoxycarbonyl, n- oder i- Propoxycarbonyl, n-, i-, s- oder t-Butoxycarbonyl, Methoxymethyl, Methoxy- ethyl, Ethoxymethyl, Ethoxyethyl, Methylthiomethyl, Methylthioethyl, Ethyl- thiomethyl, Ethylthioethyl, Carboxy, Methylaminocarbonyl, Ethylamino- carbonyl, n- oder i-Propylaminocarbonyl, Cycloproylaminocarbonyl, Cyclo- butylaminocarbonyl, Cyclopentylaminocarbonyl, Cyclohexylaminocarbonyl, Dimethylaminocarbonyl, Diethylaminocarbonyl, Ethenyl, Propenyl oder Butenyl.
- R⁴: steht bevorzugt für Methyl, Ethyl, n-Propyl, iso-Propyl, n-Butyl, iso-Butyl, tert-Butyl, n-Pentyl, Cyclopropyl, Cyclopentyl, Cyclohexyl, 2-Chlorethyl, 2,2,3,3,3-Pentafluorpropyl, 2,2,2-Trifluorethyl, 3-Bromopropyl, 2-Methoxy- ethyl, 2-Ethoxyethyl, 2-Methylthioethyl, Allyl, 2-Butenyl oder für jeweils gegebenenfalls einfach oder zweifach, gleich oder verschieden durch Fluor, Chlor, Brom, Methyl, Ethyl, iso-Propyl, Trifluormethyl, Methoxy oder Methylthio substituiertes Phenyl oder Benzyl.
- p: steht bevorzugt für 1 oder 2.
- X: steht bevorzugt für Sauerstoff.
- Y: steht bevorzugt für gegebenenfalls einfach oder zweifach, gleich oder verschieden substituiertes Methylen, wobei als Substituenten beispielhaft genannt seien: Methyl, Ethyl oder für gegebenenfalls einfach oder zweifach, gleich oder verschieden substituiertes Phenyl, wobei als Substituenten beispielhaft genannt seien: Fluor, Chlor, Methyl, Methoxy, Trifluormethyl, Cyano oder Nitro.
- Het: steht besonders bevorzugt für einen Heterocyclus aus der folgenden Gruppe von Heterocyclen:

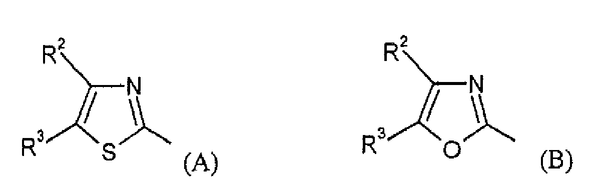

- R²: steht besonders bevorzugt für Wasserstoff.
- R³: steht bevorzugt für Wasserstoff, Fluor oder Chlor.
- p: steht besonders bevorzugt für 1.
- Y: steht besonders bevorzugt für gegebenenfalls einfach oder zweifach, gleich oder verschieden durch Methyl oder Ethyl substituiertes Methylen, oder für gegebenenfalls einfach oder zweifach, gleich oder verschieden durch Fluor, Chlor, Methyl, Methoxy, Trifluormethyl, Cyano oder Nitro substituiertes Phenyl.
- Het: steht am meisten bevorzugt den folgenden Heterocyclus:

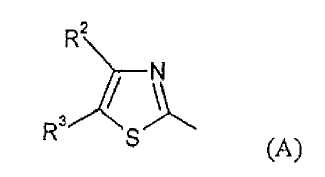

- R³: steht am meisten bevorzugt für Chlor.

Das erfindungsgemäße Verfahren lässt sich schematisch z.B. wie folgt darstellen:

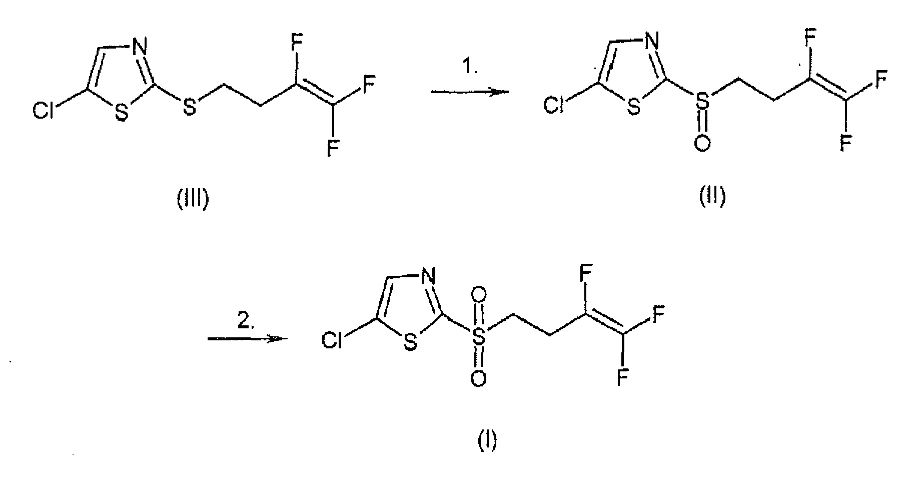

Das erfindungsgemäße Verfahren führt zu höheren Ausbeuten, wobei aus dem Reaktionsgemisch nach der Aufarbeitung das gewünschte Produkt ohne weitere Aufreinigung auskristallisiert. Eine Destillation oder Chromatographie ist nicht mehr notwendig, was sich ebenfalls vorteilhaft auf die Ausbeute auswirkt.

Die erfindungsgemäßen Verfahrensschritte können jeweils direkt aufeinander folgend oder auch jeweils einzeln durchgeführt werden, wobei das jeweilige Produkt auch gereinigt werden kann. Bevorzugt wird das erfindungsgemäß Verfahren verwendet, um ausgehend von Sulfiden der Formel (III) die Sulfone der Formel (I) herzustellen. Das erfindungsgemäße Verfahren kann ebenso zur Herstellung von Sulfoxiden der Formel (II), 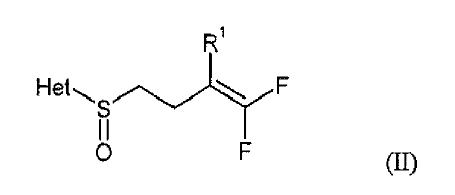 worin R¹ und Het die vorstehend angegebene Bedeutung haben,
aus Verbindungen der Formel (III) verwendet werden. Dabei muss beachtet werden, dass der pH-Wert während der Oxidation des Sulfids zum Sulfoxid bei 1 bis 3 gehalten wird.

Ebenso kann das erfindungsgemäße Verfahren zur Herstellung von Verbindungen der Formel (I) aus Verbindungen der Formel (II) verwendet werden. Hier ist ein pH-Wert von 6 bis 10, vorzugsweise von 8 bis 9 erforderlich (vgl. Herstellungsbeispiel).

Das erfindungsgemäße Verfahren erfordert für die Herstellung des Sulfons aus dem Sulfid nach der Oxidation zum Sulfoxid in einem ersten Schritt eine Erhöhung des pH-Werts, der während der Reaktion bei 6 bis 10, vorzugsweise bei 8 bis 9 liegen sollte (pH-Kontrolle). Dabei kann der pH-Wert entweder im Anschluss an die Durchführung des ersten Schrittes (Herstellung des Sulfoxids) oder auch schon während der Zugabe der ersten Portion des Kaliumsalzes bzw. des Oxone® zum Sulfid durch die Zugabe von Base erhöht werden (vgl. auch Herstellungsbeispiel).

Als Basen können alle gängigen Alkali- und Erdalkalihydroxide oder Alkali- und Erdalkalicarbonate, Amine etc. verwendet werden. Bevorzugt werden NaOH, KOH, K₂CO₃ oder Na₂CO₃ verwendet. Besonders bevorzugt wird NaOH verwendet.

Im Reaktionsansatz eventuell vorhandene Peroxide sollten aus Sicherheitsgründen während der Aufarbeitung zerstört werden. Es ist deshalb wichtig, bei der Aufarbeitung die Anwesenheit von Peroxiden zu überprüfen, z.B. mit einem Perex-Test®.

Die beim erfindungsgemäßen Verfahren verwendeten Verbindungen zur Herstellung der Verbindungen der Formel (I) und der Formel (II) sind durch die Formeln (II) und (III) allgemein charakterisiert. Die Verbindungen der Formel (II) und (III), in welchen R¹ für Fluor steht, sind bekannte Verbindungen und werden z.B. in WO 95/24403, WO 01/02378, WO 01/66529, WO 02/06259, WO 02/06257, WO 02/06256 und WO 02/06260 beschrieben und können z.B. nach den dort angegebenen Verfahren hergestellt werden. Verbindungen der Formel (II) und (III) in welcher R¹ für H steht, werden z.B. in WO 95/24403 beschrieben und können nach dem dort angegebenen Verfahren hergestellt werden.

Als bevorzugtes Salz der Peroxomonoschwefelsäure wird das Kaliumhydrogenperoxomonosulfat, KHSO₅ (CAS-RN 10058-23-8) verwendet. Das KHSO₅ wird in seiner stabilen Form als Tripelsalz der Formel 2 KHSO₅ · KHSO₄ · K2S04 (5:3:2:2) (CAS-RN 70693-62-8) eingesetzt. Insbesondere bevorzugt werden im erfindungsgemäßen Verfahren Oxone® oder Caroat®, wobei Oxone® besonders hervorgehoben wird, als Oxidationsmittel verwendet.

Oxone® ist als Oxidationsmittel für Sulfide bekannt (z.B. R. J. Kennedy, A.M. Stock, J. Org. Chem. (1960), 25, 1901; J. Leonard et al., Synthesis (1999), 507; K.S. Webb, Tetrahedron Lett. (1994), 35, 3457). So konnten z.B. Episulfide mit Oxone® zu Episulfonen oxidiert werden ("The First Preparation of Episulfones from Episulfides: Oxidation Using Oxone®/ Tetrafluoroacetone", P. Johnson and J.K. Tailor, Tetrahedron Lett. (1997) 38, 5873).

Die aktive Komponente von Oxone® ist das vorstehend genannte Kaliumhydrogenperoxomonosulfat, KHSO₅, [K^{+ -}O-S(=O)₂(-OOH)], auch bekannt als Kaliummonopersulfat, welches als Tripelsalz der Formel 2 KHSO₅ · KHSO₄ · K₂SO₄ (5:3:2:2) z.B. unter den genannten Markennamen Caroat® oder Oxone® erhältlich ist. Das Monokaliumsalz wird z.B. als Bleich- und Oxidationsmittel in Reinigungsmitteln verwendet.

Das erfindungsgemäße Verfahren zur Herstellung der Verbindungen der allgemeinen Formel (I) bzw. der allgemeinen Formel (II) werden vorzugsweise unter Verwendung von Verdünnungsmitteln durchgeführt. Als Verdünnungsmittel zur Durchführung des erfindungsgemäßen Verfahrens kommen neben Wasser vor allem wassermischbare Lösungsmittel in Betracht, z.B. Ketone, wie Aceton; Nitrile, wie Acetonitril, Propionitril oder Butyronitril; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, Carbonsäuren wie Ameisensäure, Eisessig oder deren Gemische, Ether wie Tetrahydrofuran oder Dioxan, jedoch ebenso nicht wassermischbare, inerte organische Lösungsmittel, wobei Phasentransferkatalysatoren anwesend sein müssen. Hierzu gehören aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Butanon oder Methyl-isobutyl-keton; Petrolether, Hexan, Cyclohexan, Dichlormethan, Chloroform, Tetrachlorkohlenstoff; Ether, wie Diethylether, Diisopropylether, 2-Methyltetrahydrofuran, Methyl-t-butylether oder Ethylenglykoldimethyl- oder -diethylether; Ester wie Essigsäureethylester oder Essigsäureisopropylester. Bevorzugte wassermischbare Lösungsmittel sind Acetonitril, Aceton, Alkohole, insbesondere Methanol, Ethanol, n- oder i-Propanol, Dioxan, Tetrahydrofuran oder die oben genannten Carbonsäuren. Ein bevorzugtes nicht wassermischbares Lösungsmittel ist z.B. Toluol in Anwesenheit eines Phasentransferkatalysators. Besonders bevorzugt werden als Lösungsmittel die genannten Alkohole verwendet.

Als Phasentransferkatalysatoren kommen z.B. Tetrabutylammonium-bromid, Tetrabutylammonium-chlorid, Tetraoctylammonium-chlorid, Tetrabutylammoniumhydrogensulfat, Methyl-trioctylammonium-chlorid, Hexadecyl-trimethylammoniumchlorid, Hexadecyl-trimethylammonium-bromid, Benzyl-trimethylammoniumchlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphonium-bromid, Tetrabutylphosphoniumchlorid, Tributyl-hexadecylphosphonium-bromid, Butyl-triphenylphosphoniumchlorid, Ethyl-trioctylphosphonium-bromid, oder Tetraphenylphosphonium-bromid in Betracht. Insbesondere kommen die jeweiligen Hydrogensulfate der Phasentransferkatalysatoren in Betracht.

Die Reaktion kann innerhalb eines relativen großen Temperaturbereichs durchgeführt werden, bevorzugt bei Temperaturen zwischen -20° und 150°C. Bevorzugt werden Temperaturen zwischen 0°C und 40°C verwendet. Bevorzugte Temperaturbereiche können auch dem Herstellungsbeispiel entnommen werden.

Der pH-Wert muss mit Blick auf das jeweils erwünschte Endprodukt (Sulfoxid oder Sulfon) ausgewählt werden. Für die Herstellung des Sulfoxids aus dem Sulfid sollte der pH-Wert zwischen 1 und 3 liegen. Für die Herstellung des Sulfons aus dem Sulfoxid muss der pH-Wert auf 6 bis 10 erhöht werden. Vorzugsweise liegt er bei 8 bis 9. Der pH-Wert kann auch schon während der Zugabe der ersten Portion der Oxone®-Lösung durch Zugabe von Base erhöht werden, wenn das Sulfid in das Sulfon umgesetzt werden soll.

Die erfindungsgemäßen Verfahren werden im Allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 50 bar, bevorzugt zwischen 1 und 10 bar - durchzuführen.

Bei der Durchführung des erfindungsgemäßen Verfahrens werden vorzugsweise zur Darstellung von einem Mol einer Verbindung der Formel (I) mindestens ein Mol einer Verbindung der Formel (III) und mindestens 2 Mol eines Salzes der Peroxomonoschwefelsäure verwendet. Das Oxone® kann z.B. -je nach Temperaturals eine bis zu 25-35%ige wässrige Lösung eingesetzt werden.

Die Aufarbeitung wird nach üblichen Methoden durchgeführt (vgl. die Herstellungsbeispiele).

In einer bevorzugten Ausführungsform des erfindungsgemäßen Verfahrens zur Herstellung der Verbindungen der Formel (I) und (II) wird eine Verbindung der Formel (III) in Lösungsmittel, bevorzugt in einem der vorstehend genannten Lösungsmittel, insbesondere bevorzugt einem Alkohol, nach langsamer Zugabe einer wässrigen Lösung von Kaliumhydrogenperoxomonosulfat, bevorzugt Oxone®, gerührt. Anschließend stellt man den pH-Wert vorzugsweise mit NaOH auf bevorzugt 8 bis 9 ein und gibt erneut eine wässrige Lösung von Kaliumhydrogenperoxomonosulfat, bevorzugt Oxone®, zu und rührt für geraume Zeit weiter, wobei der pH Wert weiter auf 8 bis 9 gehalten wird. Man saugt schließlich von den Salzen ab und reinigt das gewünschte Produkt nach üblichen Methoden auf.

Die Herstellung von Verbindungen der Formel (I) bzw. von Verbindungen der Formel (II) geht auch aus dem nachfolgenden Beispiel hervor, das die vorstehend beschriebenen Verfahrensschritte weiter illustriert. Das Beispiel ist jedoch nicht in einschränkender Weise zu interpretieren.

### Beispiele

### Beispiel 1

### Darstellung von 5-Chlor-2-[(3,4,4-trifluor-3-butenyl)sulfonyl]-1,3-thiazol

Man legt 26 g (0,1 mol) 5-Chlor-2-[(3,4,4-trifluor-3-butenyl)sulfanyl]-1,3-thiazol in 250 ml Methanol vor und tropft unter Rühren bei 5°C eine Lösung von 33,9 g Oxone® in 125 ml Wasser innerhalb von 30 Minuten hinzu. Anschließend wird die weiße Suspension bei 20°C 1,5 Stunden lang gerührt. Zur Vervollständigung der ersten Reaktionsstufe (Herstellung des Sulfoxids) gibt man nochmals 1,7 g Oxone® hinzu und rührt weitere 30 Minuten.

Anschließend kühlt man wieder auf 5°C ab, stellt mit 4 N NaOH einen pH von 8-9 ein und tropft innerhalb von 30 Minuten eine Lösung von 33,9 g Oxone in 125 ml Wasser hinzu, wobei der pH weiterhin bei 8-9 gehalten wird. Der gesamte Verbrauch an 4 N NaOH beträgt ca. 70 ml (2,54 Aquivalente bezogen auf Oxone®). Anschließend rührt man unter pH-Kontrolle 60 Minuten bei 20°C, gibt nochmals 1 g Oxone® hinzu und rührt weitere 20 Minuten.

Man saugt von den Salzen ab, wäscht den weißen Rückstand zweimal mit je 30 ml Methanol, rührt das Filtrat mit 25 ml Natriumbisulfitlösung und prüft auf Peroxide. Dann wird vom Filtrat der Methanolanteil im Vakuum abdestilliert. Vom wasserhaltigen, zweiphasigen Rückstand wird die organische Phase abgetrennt und die wässrige Phase nochmals dreimal mit Essigester extrahiert. Die vereinigten organischen Phasen werden auf Peroxid getestet, mit Natriumsulfat getrocknet und eingedampft. Man erhält 27,6 g (92,2 % der Theorie) eines gelblichen Öls, das nach Anreiben und Abkühlen kristallisiert und einen Schmelzpunkt von 34 °C besitzt. Der Gehalt (HPLC gegen Standard) beträgt 97,6 %.

## Patentansprüche

1. Verfahren zur Herstellung von Verbindungen der Formeln (I) und (II), 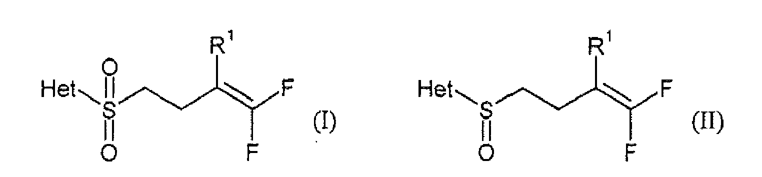 worin
R¹ für Wasserstoff oder Fluor steht, und
Het für einen Heterocyclus aus der nachfolgenden Gruppe von Heterocyclen steht, 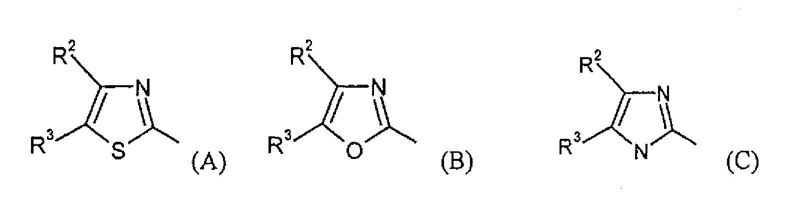 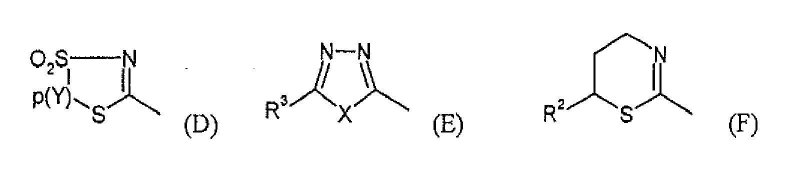 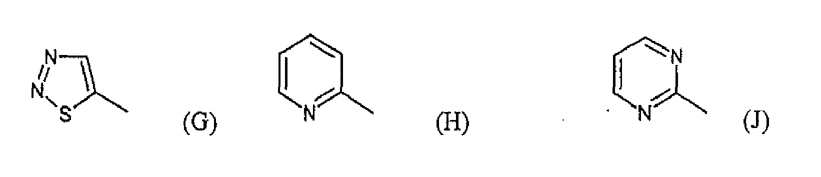 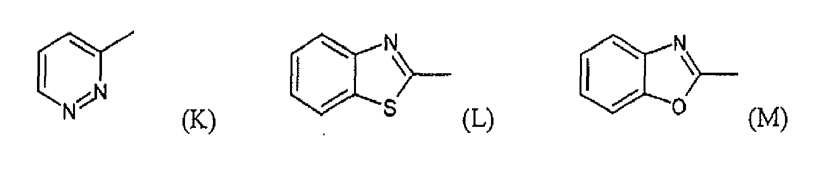 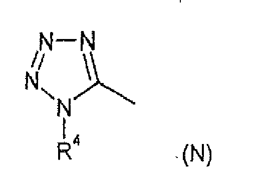
worin
R² für Wasserstoff, Halogen, C₁-C₄-Alkyl oder C₁-C₄-Halogenalkyl steht,
R³ für Wasserstoff, Halogen, sowie jeweils gegebenenfalls durch Halogen, Methyl, Ethyl, n- oder i-Propyl oder n-, i-, s- oder t-Butyl, Methoxy, Ethoxy, n- oder i-Propoxy oder n-, i-, s- oder t-Butoxy substituiertes C₁-C₄-Alkyl, C₁-C₄-Alkoxy, C₁-C₄-Alkylthio, C₁-C₄- Alkylsulfinyl, C₁-C₄-Alkylsulfonyl, C₁-C₄-Alkoxy-carbonyl, C₁-C₄- Alkoxy-C₁-C₄-alkyl, C₁-C₄-Alkylthio-C₁-C₄-alkyl, Carboxy, C₁-C₄- Alkylamino-carbonyl, C₃-C₆-Cycloalkyl-aminocarbonyl, C₁-C₄- Dialkyl-aminocarbonyl, C₂-C₄-Alkenyl, C₂-C₄-Alkenylthio, C₂-C₄- Alkenylsulfinyl oder C₂-C₄-Alkenylsulfonyl steht,
R⁴ für C₁-C₈-Alkyl, C₂-C₆-Alkenyl, C₁-C₄-Haloalkyl, C₁-C₄-Alkoxy- C₁-C₄-alkyl, C₁-C₄-Alkylthio-C₁-C₄-alkyl, C₃-C₈-Cycloalkyl oder für jeweils gegebenenfalls durch Halogen, C₁-C₄-Alkyl, C₁-C₄-Alkoxy, C₁-C₄-Alkylthio oder C₁-C₄-Haloalkyl substituiertes Phenyl oder Benzyl steht,
p für 1, 2 oder 3 steht,
X für Sauerstoff oder Schwefel steht, und
Y für gegebenenfalls einfach oder zweifach, gleich oder verschieden substituiertes Methylen steht, wobei als Substituenten beispielhaft ge- nannt seien: jeweils gegebenenfalls durch Halogen, C₁-C₄-Alkoxy, C₁- C₄-Alkylthio, C₁-C₄- Halogenalkoxy oder C₁-C₄-Halogenalkylthio substituiertes C₁-C₄-Alkyl, C₂-C₄-Alkenyl oder C₂-C₄-Alkinyl, sowie für gegebenenfalls einfach bis dreifach, gleich oder verschieden substituiertes Phenyl, wobei als Substituenten beispielhaft genannt seien: Halogen, Cyano, Nitro, C₁-C₄-Alkyl, C₁-C₄-Alkoxy, C₁-C₄- Alkylthio, C₁-C₄-Halogenalkyl, C₁-C₄-Halogenalkoxy oder C₁-C₄- Halogenalkylthio,
indem man eine Verbindung der Formel (III), 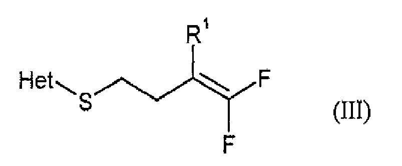 worin
R¹ und Het die vorstehend genannte Bedeutung haben,
mit einem Salz der Peroxomonoschwefelsäure, H₂SO₅,
gegebenenfalls in Gegenwart eines Reaktionshilfsmittels und gegebenenfalls in Gegenwart eines Verdünnungsmittels reagieren lässt.

2. Verfahren zum Herstellen von Verbindungen der Formel (I) gemäß Anspruch 1, **dadurch gekennzeichnet, dass** man Verbindungen der Formel (II) gemäß Anspruch 1 mit einem Salz der Peroxomonoschwefelsäure, H₂SO₅, gegebenenfalls in Gegenwart eines Reaktionshilfsmittels und gegebenenfalls in Gegenwart eines Verdünnungsmittels reagieren lässt.

3. Verfahren gemäß Anspruch 2, **dadurch gekennzeichnet, dass** das Verfahren bei einem pH-Wert von 6 bis 10 durchgeführt wird.

4. Verfahren zum Herstellen von Verbindungen der Formel (II) gemäß Anspruch 1, **dadurch gekennzeichnet, dass** man Verbindungen der Formel (III) gemäß Anspruch 1 mit einem Salz der Peroxomonoschwefelsäure, H₂SO₅, gegebenenfalls in Gegenwart eines Reaktionshilfsmittels und gegebenenfalls in Gegenwart eines Verdünnungsmittels reagieren lässt.

5. Verfahren gemäß Anspruch 4, **dadurch gekennzeichnet, dass** das Verfahren bei einem pH-Wert von 1 bis 3 durchgeführt wird.

6. Verfahren gemäß einem der Ansprüche 1 bis 5, **dadurch gekennzeichnet, dass** es sich bei dem Salz der Peroxomonoschwefelsäure um Kaliumhydrogenperoxomonosulfat (2 KHSO₅ · KHS04 . · K₂SO₄ (5:3:2:2)), bevorzugt um Oxone® oder Caroat® handelt.

7. Verfahren gemäß einem der Ansprüche 1 bis 6, **dadurch gekennzeichnet, dass** die Reaktion bei einer Temperatur von -20°C bis 150°C durchgeführt wird.

8. Verfahren gemäß einem der Ansprüche 1 bis 7, **dadurch gekennzeichnet, dass**
R¹ für Fluor steht,
Het für einen Heterocyclus aus der nachfolgenden Gruppe von Heterocyclen steht, 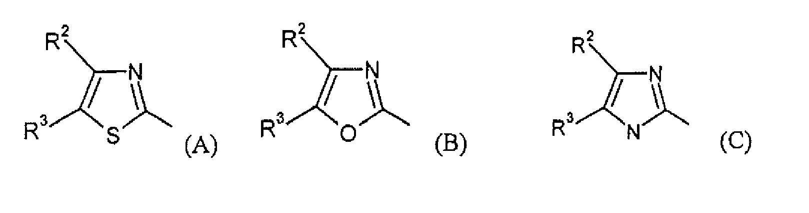 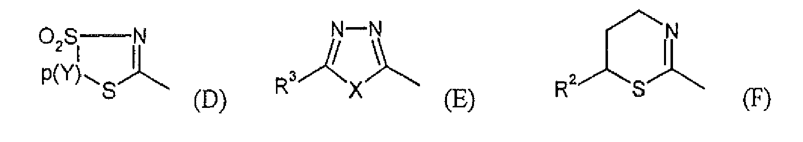 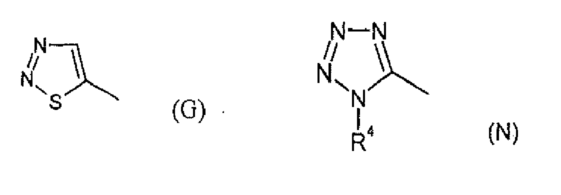
R² für Wasserstoff, Fluor oder Chlor steht,
R³ für Wasserstoff, Fluor, Chlor, sowie jeweils gegebenenfalls durch Fluor, Chlor, Methyl, Ethyl, n- oder i-Propyl oder n-, i-, s- oder t- Butyl, Methoxy, Ethoxy, n- oder i-Propoxy oder n-, i-, s- oder t- Butoxy substituiertes Methyl, Ethyl, n- oder i-Propyl, n-, i-, s- oder t- Butyl, Methoxy, Ethoxy, n- oder i-Propoxy, n-, i-, s- oder t-Butoxy, Methylthio, Ethylthio, n-, oder i-Propylthio, n-, i-, s- oder t-Butylthio, Methylsulfinyl, Ethylsulfinyl, Methylsulfonyl, Ethylsulfonyl, Methoxycarbonyl, Ethoxycarbonyl, n- oder i-Propoxycarbonyl, n-, i-, s- oder t-Butoxycarbonyl, Methoxymethyl, Methoxyethyl, Ethoxy- methyl, Ethoxyethyl, Methylthiomethyl, Methylthioethyl, Ethylthio- methyl, Ethylthioethyl, Carboxy, Methylaminocarbonyl, Ethylamino- carbonyl, n- oder i-Propylaminocarbonyl, Cycloproylaminocarbonyl, Cyclobutylaminocarbonyl, Cyclopentylaminocarbonyl, Cyclohexyl- aminocarbonyl, Dimethylaminocarbonyl, Diethylaminocarbonyl, Ethe- nyl, Propenyl oder Butenyl steht,
R⁴ steht bevorzugt für Methyl, Ethyl, n-Propyl, iso-Propyl, n-Butyl, iso- Butyl, tert-Butyl, n-Pentyl, Cyclopropyl, Cyclopentyl, Cyclohexyl, 2- Chlorethyl, 2,2,3,3,3-Pentafluorpropyl, 2,2,2-Trifluorethyl, 3- Bromopropyl, 2-Methoxyethyl, 2-Ethoxyethyl, 2-Methylthioethyl, Allyl, 2-Butenyl oder für jeweils gegebenenfalls einfach oder zweifach, gleich oder verschieden durch Fluor, Chlor, Brom, Methyl, Ethyl, iso-Propyl, Trifluormethyl, Methoxy oder Methylthio substituiertes Phenyl oder Benzyl steht,
p für 1 oder 2 steht,
X für Sauerstoff steht, und
Y für gegebenenfalls einfach oder zweifach, gleich oder verschieden substituiertes Methylen steht, wobei als Substituenten beispielhaft genannt seien: Methyl, Ethyl oder für gegebenenfalls einfach oder zweifach, gleich oder verschieden substituiertes Phenyl, wobei als Substituenten beispielhaft genannt seien: Fluor, Chlor, Methyl, Methoxy, Trifluormethyl, Cyano oder Nitro.

9. Verfahren gemäß einem der Ansprüche 1 bis 8, **dadurch gekennzeichnet, dass**
Het für einen Heterocyclus aus der folgenden Gruppe von Heterocyclen steht, 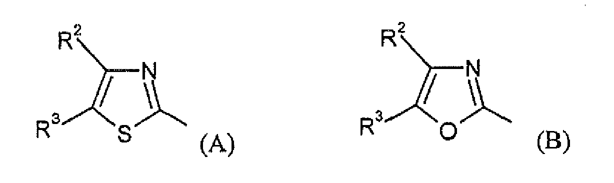
R² für Wasserstoff steht, und
R³ für Wasserstoff, Fluor oder Chlor steht.

10. Verfahren gemäß einem der Ansprüche 1 bis 9, **dadurch gekennzeichnet, dass**
Het für den folgenden Heterocyclus steht, 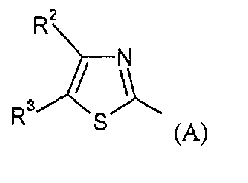
R² für Wasserstoff steht, und
R³ für Chlor steht.
